Europäisches Patentamt

European Patent Office

Office européen des brevets

(11) Publication number: **0 225 162 B1**

(12) **EUROPEAN PATENT SPECIFICATION**

(45) Date of publication of patent specification: **22.01.92**

(51) Int. Cl.⁵: **A61K 9/22**, A61K 9/50, A61K 9/52

(21) Application number: **86309202.9**

(22) Date of filing: **26.11.86**

(54) Inhibition of post-surgical adhesion formation by the topical administration of non-steroidal anti-inflammatory drug.

(30) Priority: **27.11.85 US 802545**
      **25.08.86 US 900122**

(43) Date of publication of application:
      **10.06.87 Bulletin  87/24**

(45) Publication of the grant of the patent:
      **22.01.92 Bulletin  92/04**

(84) Designated Contracting States:
      **BE CH DE FR GB GR IT LI NL SE**

(56) References cited:
      **EP-A- 0 102 265**
      **FR-A- 2 126 270**
      **FR-A- 2 344 290**
      **FR-A- 2 491 351**
      **US-A- 4 346 108**

      **Rote Liste 1985, item 05428**

(73) Proprietor: **ETHICON INC.**
      **U.S. Route 22**
      **Somerville New Jersey 08876(US)**

(72) Inventor: **Sheffield, Warren D.**
      **RD 3, Box 67, Blossom Hill Rd.**
      **Lebanon, NJ(US)**
      Inventor: **Johns, Douglas B.**
      **RD 4, Box 32**
      **Milford, NJ(US)**
      Inventor: **Shalaby, Shalaby W.**
      **328A Longview Road, RD 2**
      **Lebanon, NJ(US)**
      Inventor: **Dizerega, Gere S.**
      **420 San Juan Place, RD 2**
      **Pasadena, CA(US)**
      Inventor: **Richer, Leroy L.**
      **6924 Willard Avenue N.**
      **San Gabriel, CA(US)**

(74) Representative: **Jones, Alan John et al**
      **CARPMAELS & RANSFORD 43 Bloomsbury Square**
      **London, WC1A 2RA(GB)**

## Description

The present invention relates to the inhibition of post-surgical adhesion formation.

Adhesion formation is a major post-surgical complication with no practical solution. The incidence of adhesion formation following surgery approaches l00 per cent, according to some sources, with a clinically significant complication rate of about 5 to l0 per cent, depending on the type of surgery. Among such complications are bowel obstruction, infertility, and pain. Occasionally, adhesions necessitate a second operative procedure to remove the adhesion, which may in turn further aggravate the problem.

Because of the seriousness of the problem, much medical research has been performed in efforts to find ways to combat adhesions. See, for instance, Stangel et al., "Formation and Prevention of Postoperative Abdominal Adhesions", the Journal of Reproductive Medicine, Vol. 29, No. 3, March l984 (pages l43-l56), and DiZerega, "The Cause and Prevention of Postsurgical Adhesions", published by Pregnancy Research Branch, National Institute of Child Health and Human Development, National Institutes of Health, Building l8, Room l0l, Bethesda, MD 20205.

Among the approaches that have been tried for preventing post-surgical adhesion are the following:

Systemic administration of ibuprofen (e.g., see Singer, U.S. Patent No. 4,346,l08):

Parenteral administration of antihistamines, corticosteroids, and antibiotics;

Intraperitoneal administration of dextran solution and of polyvinylpyrrolidone solution; and

Systemic administration of oxyphenbutazone, a non-steroidal anti-inflammatory drug that acts by inhibiting prostaglandin production.

Corticosteroids have been administered intraperitoneally as well as systemically in efforts to prevent adhesions. (See the Stangel et al. article, cited above, on page l47, as well as the articles cited therein.) Some studies have questioned the efficacy of corticosteroids in adhesion prevention. In high doses, these materials may suppress the immune system and interfere with wound healing. Therefore, the use of corticosteroids does not seem to be an acceptable solution to the post-operative adhesion problem.

On the basis of the results of animal studies and limited human clinical studies, the systemic administration of non-steroidal anti-inflammatory agents such as ibuprofen (usually in combination with other medicaments such as antibiotics) appears to be the most efficacious pharmacological means now known to reduce the incidence of post-surgical adhesions. An objection to this means is that relatively large amounts of the drug must be administered over a period of several days, thereby subjecting the patient to the significant risk of experiencing adverse side effects. Also, this means has been shown to be effective only in a limited number of types of surgical procedures, e.g., gynecological surgery. As reported by Nishimura, Nakamura, and diZerega (Journal of Surgical Research 36, ll5-l24, February l984), the minimum effective dose of systemically administered ibuprofen to inhibit postsurgical adhesion formation after abrasion or ischemia of the uterine horn of rabbits, is 70 mg/kg/day, administered once a day for at least 3 and preferably for 5 days post-operatively, with an additional dose l hour before surgery. In a similar series of experiments, Siegler et al. (Fertility and Sterility 34, No. l, July l980, pages 46-49) found an effective dose of systemically administered ibuprofen to be about 2l mg/kg/day, administered three times daily (in three 7 mg/kg doses) for two days post-operatively, with the initial injection being given 30 minutes before surgery. The authors also reported that the best results were found in two rabbits that were each inadvertently given three extra 7 mg/kg doses. In order to deter adhesion formation in surgery to try to cure infertility, Corson et al. (The Journal of Reproductive Medicine, Vol. 29, No. 3, pages l43-l56, March l984) recommend a regimen including systemically administered ibuprofen, 400 mg per dose three to four doses per day, starting the night of surgery and continuing to the fifth postoperative day. Assuming that the average woman weighs about 48 kg (ll0 pounds), this is a recommended dosage of 25 to 33 mg/kg/day. Singer, in U.S. Patent No. 4,346,l08, recommends a dosage of from about 2.5 to 50 mg/kg/day in single or divided doses, for ibuprofen administered systemically to combat adhesions.

The Singer patent, and the journal articles by Nishimura et al., Siegler et al., and Corson et al., all cited above, disclose the systemic administration of ibuprofen to combat adhesion formation.

Lenk et al., U.S. Patent No. 4,522,803, at column 17, lines 67 et seq. disclose phospholipid vesicles containing anti-inflammatory agents. Only steroid anti-inflammatory agents are specifically disclosed. No anti-adhesion utility is disclosed.

Systemic administration of oxyphenbutazone to combat adhesions has been proposed. See, for example, Kapur et al., "Prevention of Reformation of Peritoneal Adhesions", Arch. Surg., Vol. 105, Nov. 1972, Pages 761-764.

The present invention provides a pharmaceutical composition for topical application comprising a topically effective amount of a non-steroidal anti-inflammatory drug (NSAID) or a pharmaceutically acceptable salt or ester thereof as the active ingredient in a topically applicable pharmaceutically acceptable carrier, provided that the NSAID is not aspirin wherein said carrier is a controlled release carrier which releases the active ingredient in an effective amount over a period of time of from about one to seven days.

The present invention also provides a pharmaceutical composition adapted for topical administration in a single dose comprising a topically effective amount of a non-steroidal anti-inflammatory drug (NSAID) or a pharmaceutically acceptable salt or ester thereof as the active ingredient in a topically applicable pharmaceutically acceptable carrier suitable for topical adminstration to a site of surgical trauma, provided that the NSAID is not aspirin and wherein the concentration of active ingredient in said composition is from 0.025 to 5 milligrams per millilitre. Also this invention provides a pharmaceutical composition adapted for topical administration continually over a period of time comprising a topically effective amount of a non-steroidal anti-inflammatory drug (NSAID) or a pharmaceutically acceptable salt of ester thereof as the active ingredient in a topically applicable pharmaceutically acceptable carrier suitable for topical administration to a site of surgical trauma, provided that the NSAID is not aspirin and wherein the concentration of active ingredient in said composition is from 0.01 to 10 milligrams per millilitre.

The present invention also provides the use of a non-steroidal anti-inflammatory drug (NSAID) or a pharmaceutically acceptable salt or ester thereof in the production of a topically applicable medicament for topical use in inhibiting the formation of post-surgical adhesions in mammals, provided that the NSAID is not aspirin.

The pharmacologically active compounds that are employed in this invention are the non-steroidal anti-inflammatory drugs such as ibuprofen, tolmetin, indomethacin, sulindac, suprofen, oxyphenbutazone, and pharmaceutically acceptable salts or esters thereof. NSAID's comprise a recognized class of compounds.

In use, the active agent is applied topically to the site of surgical trauma in effective amounts for at least two and up to about seven days after the operation, to thereby inhibit the formation of post-surgical adhesions. By the term "topically", is meant that the NSAID is administered non-systemically to the surface of the tissue (internal or, in some cases, external) to be treated, for local effect. The term "site of surgical trauma" is meant to include the site of tissue that has been injured in any way, and includes, for example, tissue sites that have undergone incision, excision, abrasion, contusion, laceration, anastomosis, manipulation, prosthetic surgery, curettage, orthopedic surgery, neurosurgery, cardiovascular surgery, or plastic or reconstructive surgery. "Site of surgical trauma" also includes tissue that is adjacent to the injured tissue.

The compositions of the invention are useful in any surgical procedure in which it is desired to inhibit the formation of post-surgical adhesions. They are thus broadly useful in all types of surgery in which adhesion formation can be complication.

The NSAID may be administered to the site of surgical trauma by any convenient mode such as, for example, by lavage, by catheter, by coating directly on the site in a salve, ointment, gel, cream, aqueous surface active composition, emulsion, suspension, or foam, or by any other convenient mode. The site can be contacted directly, as by applying a salve, or in some cases the medicament can be introduced to a site near the site of trauma and natural migration of fluids will serve to carry the medicament to the desired site. Such natural migration of fluids can occur, for instance, intraperitoneally, in response to peristaltic contraction of the intestines.

The NSAID is ordinarily administered in a sterile formulation in a pharmaceutically acceptable carrier or vehicle such as phosphate buffered saline ("PBS"), isotonic saline, purified water, an organic carrier such as a lipid, for example, a phospholipid micelle or vesicle, dextran, polymers (especially p-dioxanone, lactide, and/or glycolide based absorbable polymers), which may be in the form of microcapsules or which may be incorporated in a salve- or ointment-like formulation, or in a aqueous solution of a surfactant such as a polyoxyethylene-polyoxypropylene block copolymer of a sorbitan fatty acid ester-polyoxyethylene ether. Suitable absorbable polymers include a homopolymer or copolymer of lactic acid, glycolic acid, their cyclic dimer esters, or p-dioxanone. Such polymers are preferably in the form of microcapsules. These microcapsules may include lecithin in an amount sufficient to enhance the ability of said microcapsules to adhere to the site of surgical trauma.

Sterilization of the formulation may be accomplished in the usual ways, including aseptic preparation, filtration, exposure to gamma radiation, autoclaving, and the like. According to one embodiment of the invention, the NSAID is contained in a controlled release carrier that is capable of releasing the active drug for a period of at least one, preferably at least two, and up to about seven days. The mode of delivery of the NSAID is preferably continually over the critical wound healing period, such as will be the case when the drug is applied to the site of surgical trauma in a single dose via a controlled release carrier, or continually via a catheter.

A general procedure for preparing a polymeric microcapsule containing a drug, and which is applicable to incorporating NSAID's in polymeric microcapsules, is the following:

1. The drug and the polymer are dissolved in a volatile organic solvent:

2. The solvent containing the drug and polymer is dispersed in water with a dispersing agent:

3. The organic solvent is evaporated from the dispersion product of step 2, either by mild heating or by vacuum evaporation, or by a combination of the two; and

4. The resulting microcapsules are recovered from the aqueous dispersion by customary procedures such as filtration, centrifugation, or the like, usually coupled with one or more washing steps.

This procedure is illustrated for this invention by Examples 1 2, 9, and 10, below:

Example 1

(a) To a small vial was added 9.39 grams of poly(lactide-co-glycolide-65:35) (a 65/35, by weight, copolymer of lactide and glycolide having an inherent viscosity in chloroform of 0.5 dl/g), 0.75 gram refined sesame seed oil, 1.140 grams ibuprofen, and 40 milliliters of dichloromethane. The solution obtained was added to 400 milliliters of aqueous 5% wt/vol poly(vinyl alcohol) (Air Products Vinol Grade 523) in a one liter resin kettle equipped with a mechanical stirrer and vacuum take off, which was being cooled in an ice/water bath and stirred at 500 rpm. After allowing 10 minutes for emulsification, vacuum was slowly applied by means of an aspirator to an absolute pressure of 66.66 kPa (500 mm of mercury) over 1.5 hours. The vacuum was then maintained for an additional 19.5 hours (to remove the dichloromethane solvent), at which time vacuum and stirring was stopped and the contents of the flask was poured into a one liter beaker and diluted to 800 milliters total volume with water. (An alternative method for removing the dichloromethane solvent is to heat at 30°C. for two hours at atmospheric pressure.) The contents of the beaker were added to centrifuge tubes and they were centrifuged at about 1000 rpm for two minutes. The liquid was decanted, fresh water was added, and centrifugation was repeated. This procedure was repeated one more time, and after the third wash, the last traces of water were removed by freeze drying. The dried sample was a free flowing white powder which weighed 2.03 grams. The powder was examined at 100% and was found to contain microcapsules which ranged in size from about 1 to 10 microns. No free drug crystals were noted. Subsequent NMR analysis indicated that 8.6 wt. % ibuprofen was present.

(b) To make control microcapsules, the foregoing procedure is repeated without adding the ibuprofen.

(c) Microcapsules produced as described above, both with and without ibuprofen, are dispersed in an aqueous dispersion of lecithin in proportions of 1.5 grams of microcapsules per 50 milliliters of 0.05 weight per cent aqueous lecithin. After freeze drying, 1.4 grams of microcapsules are obtained which contain about 1.4 weight per cent of lecithin.

Example 2

To a small vial was added 1.50 grams of poly(lactide-co-glycolide - 65:35), 0.50 gram ibuprofen, and 5 milliliters of dichloromethane. The resulting solution was added to 50 milliliters of 3% aqueous poly(vinyl alcohol) solution which was being cooled in an ice/water bath and stirred at 500 rpm. Vacuum was applied as before and after washing and freeze-drying, 1.100 grams of microcapsules was obtained. The microcapsules ranged in size from 10 to 120 microns and were found to contain 17.1% by weight ibuprofen by NMR.

Example 3

Ibuprofen/poly(lactide-co-glycolide-65:35) microcapsules (Example 2) which were found to contain 17.1% by weight ibuprofen (NMR) were investigated. In five separate 113.4 g (4 ounce) amber jars was placed 100 milligrams of microcapsules and 100 milliliters of pH 7.27 phosphate buffer. The caps were tightly closed and the jars incubated at 37°C., with no agitation. After 15 minutes, 1 day, 2 days, 7 days, and 14 days, a jar was removed, the microcapsules collected by filtration, washed well with water, dried under vacuum, and analyzed for ibuprofen content by NMR. The results shown in the attached table indicate that approximately 50% of the drug was released in the first 7 days and the remainder was completely released by day 14. (Negligible polymer weight loss occurred over the 14 day period.)

Table I

| In-Vitro Release of Ibuprofen From Poly(lactide-co-glycolide) Microcapsules | | |
|---|---|---|
| Sample | Time (Days) | WT % IBF REMAINING (% of TOT CAPSULE WT) |
| 1 | 0 | 17.0 |
| 2 | 1 | 14.3 |
| 3 | 2 | 13.6 |
| 4 | 7 | 10.0 |
| 5 | 14 | 0.0 |

Methods for incorporating drugs in lipid carriers are known in the art. For instance, one procedure for encapsulating a drug in a phospholipid vesicle is the following:

a lipid or mixture of lipids such as lecithin or other phospholipid, which may be mixed with cholesterol or other lipoid substance, is dissolved in an organic solvent such as diethyl ether; and

an aqueous phase containing the material to be encapsulated (in this case, an NSAID) is added to the lipid solution, and the mixture is agitated as by exposing it to ultrasonic sound waves (sonicated). Preferably, the organic solvent is removed during sonication, as by use of heat or vacuum or both, although in some cases the solvent can be removed after the sonication. This procedure typically produces a unilamellar vesicle.

Another procedure for producing a phospholipid vesicle (in this case a multilamellar vesicle "MLV") containing a medicament is to form a film of dry lipid, as by evaporating the solvent from an organic solvent solution containing a lipid to form a film on the walls of the vessel containing the solution, and then stirring in the aqueous phase containing the NSAID to be encapsulated. (The evaporation can be done by spray drying or by vacuum evaporation, or by any other convenient method.) Free unencapsulated NSAID can be separated from MLV's by centrifugation at, e. g., 12,000 rpm.

Preferably, the vesicle containing the NSAID is dehydrated, as by freeze drying, after preparation, in order to insure long term storage stability. The aqueous vesicle suspension can be reconstituted just prior to use by adding sterile phosphate buffered saline, sterile water, or the like.

The use of multilamellar vesicles of comparatively large size (that is, greater than l micron, e. g., from about l to about l0 microns) appears to be preferable in order to increase the dwell time of the vesicle containing the NSAID in the peritoneal cavity (or other body cavity). It is also preferred to used a pure or synthetic phosphatidylcholine in which the fatty acid moieties in the phosphatidylcholine molecule are derived from a single fatty acid, in preparing the vesicle instead of natural lecithin, which is ordinarily a mixture of compounds. Example l3, below, illustrates the preparation of a multilamellar vesicle containing an NSAID.

The following United States patents describe the preparation, by various procedures, of phospholipid vesicles containing various medicaments:

Lenk et al. No. 4,522,803

Baldeschwieler et at. No. 4,3l0 505

Mezei et al. No. 4,485,054

Gersonde et al. No. 4,452,747

Kelly No. 4,356,l67

Papahadjopoulos et al. No. 4,24l,046

Suzuki et al. No. 4,0l6,l00

Sache et al. No. 4,239,754

MacDonald No. 4,532,089

Rahman et al. No 3,993,754

See also Callahan et al., European Patent Application No. 0l26580, published November 28, l984, and Gregoriadis, "The Carrier Potential of Liposomes In Biology and Medicine", New England Journal of Medicine, Vol. 295, pp. 704-7l0 and pp. 765-770 (Sept. 23 and 30, l976).

The foregoing are general procedures which can be utilized for the incorporation of NSAID's in liposomes.

Other procedures for containing drugs in phospholipids (micelles or liposomes), and which are applicable to NSAID's, are described in Sears, U.S. Patents Nos. 4,426,330 and 4,l45,4l0, and Sears et al., U.S. Patent No. 4,298,594.

It is not essential that the NSAID medicament used in the composition of the invention be encapsulated in an inside compartment or compartments of the carrier as will normally be the case when the carrier is a

EP 0 225 162 B1

phospholipid vesicle. In some cases it is acceptable for the NSAID to be dissolved or otherwise distributed more or less evenly throughout the carrier.

The non-steroidal anti-inflammatory drug is delivered to the site of surgical trauma in effective quantities over the critical wound healing period (which period varies from patient to patient and with the type of surgical trauma encountered, but is usually from about two to five days, and in some cases up to seven days or more, post operatively). The examples below illustrate the order of magnitude of effective quantities of the drug and the period of time over which the drug is administered for effective results.

The following studies use rabbit models to illustrate the adhesion inhibition effectiveness of the topical administration of a non-steroidal anti-inflammatory drug to the site of surgical trauma:

Example 4

New Zealand white female rabbits (I.8-2.0 kg) underwent midline laparotomy using acelepromazine and ketamine anaesthesia. A $3 \times 5$ centimeter abrasion was produced over the right-lateral peritoneal side-wall by scraping the surface peritoneum with a scalpel until punctate bleeding developed over the entire $3 \times 5$ centimeter area. A second abrasion covering the same total area (I5 cm$^2$) using the same technique was developed I.5-2.0 centimeters inferior to the initial site along the right-lateral peritoneal side-wall. This second site was used as an untreated control. The serosal surface of the large bowel adjacent to the peritoneal abrasion sites was also similarly abraded.

Ibuprofen contained in poly(lactide-co-glycolide-65:35) microcapsules produced as described in Example I, were suspended in I5 weight per cent aqueous poly(vinyl pyrrolidone) "PVP" (GAF Povidone C-I5). The proportions were I5 grams of microcapsules per I00 grams of the PVP solution.

The lecithin-containing microcapsules were used in half of the experiments. The suspension was dripped on the wound in an amount such that about 40 milligrams of ibuprofen (about 470 milligrams of microcapsules) was applied to the wound site. One control was the PVP solution containing ibuprofen-free microcapsules, and the other was the PVP solution containing ibuprofen-free microcapsules with lecithin.

Seven days after the day of abrasion, the rabbits were sacrificed by pentobarbital overdose. The extent of adhesions was evaluated as follows:

1. No adhesions
2. Filmy adhesions (separable)
3. Mild adhesions (not separable - covering up to about 35% of the test area)
4. Moderate adhesions (not separable - covering about 35 to 60 % of the test area)
5. Severe adhesions (not separable - covering greater than about 60% of the test area)

The evaluation ratings set forth above are useful in the context of comparing the efficacy of various means for inhibiting the formation of adhesions. However, it should be mentioned that ultimately only a rating of "1" can be considered to be completely acceptable. Clinical complications can result from even mild adhesions, although such complications are considered to be more likely to occur with severe adhesions than with mild or moderate adhesions.

The following Table II presents the results:

6

## Table II

| Rabbit No. | Ibuprofen | Lecithin | Evaluation | |
| | | | Ibuprofen Treatment | Untreated Controls |
|---|---|---|---|---|
| 1 | yes | no | 4 | 4 |
| 2 | yes | no | 4 | 5 |
| 3 | yes | no | 1 | 1 * |
| 4 | yes | yes | 3 | 5 |
| 5 | yes | yes | 4 | 5 |
| 6 | yes | yes | 3 | 5 |
| 7 | no | no | 5 | 5 |
| 8 | no | no | 5 | 5 |
| 9 | no | no | 5 | 5 |
| 10 | no | yes | 5 | 5 |
| 11 | no | yes | 5 | 5 |
| 12 | no | yes | 5 | 5 |

----------------------------------------------------------------

* The surgical procedure followed in this experiment induces adhesion formation in about 87 per cent of untreated rabbits. Evidently this rabbit was one of the approximately 13 per cent that do not develop adhesions.

----------------------------------------------------------------

The results indicate some anti-adhesion activity exhibited by the microcapsules containing ibuprofen.

It is relevant to note that when the vehicle control site is in the same rabbit as the test site, migration of fluid in the peritoneal cavity can carry some of the medicament from the NSAID treatment site to the vehicle control site. Therefore, it is possible that the untreated (by "untreated" is meant no active medicament) control sites of rabbits Nos. l-6 in Table II and analogous untreated control sites reported below could have received small amounts of NSAID owing to migration or circulation of fluid within the peritoneal cavity. However, if any of the untreated control sites did receive some of the active medicament by such fluid migration, it would have been significantly less than that received at the treatment site in the same rabbit. Therefore, differences in results between the treatment and the control sites in the same rabbit can confidently be interpreted as being caused by the adhesion inhibition effect of the NSAID.

Example 5

In order to carry out dose response studies and time response studies, miniature osmotic pumps (Alzet mini pumps, model 2MIL or model 2002 - these pumps are described in Higuchi et al., U.S. Patent No. 3.995,631) were used to deliver the anti-adhesion agent in a continuous stream at a very low, controlled flow rate, to the site of the surgical trauma in the test rabbits over a period of time, up to seven days. The mini-pumps therefore deliver the medicament in a manner analogous to a catheter delivery mode.

New Zealand white female rabbits (1.8 to 2.0 kg) underwent midline laparotomy using acelepromazine and ketamine anaesthesia. A 3 x 5 cm flap of parietal peritoneum (about 1 mm thick) was sharply dissected from the right lateral peritoneal side-wall. The serosal surface of the adjacent large bowel was abraided with a scalpel until punctate bleeding developed. This area between the excised parietal peritoneum and adjacent large bowel serosa was then used for evaluating the efficacy of the test medicament for adhesion inhibition. A second excision of parietal peritoneum covering the same total area (about 15 cm$^2$) was performed 1.5 to 2.0 cm inferior to the initial test site along the right lateral peritoneal side-wall. Abrasion of the adjacent large bowel serosa was performed as described above for the treatment site. This second area was used to determine the effectiveness of the surgical procedure in producing adhesions and the response to vehicle controls.

Alzet mini pumps containing ibuprofen dissolved in phosphate buffered saline ("PBS") were sewn into the right dorsal subcutaneous space of the test rabbit with VICRYL (Polyglactin 910) sutures placed 3 to 5 millimeters from each end of the pump. The polyethylene catheter tip leading from the pump into the peritoneal cavity of each rabbit was placed 2 to 3 millimeters over the injury test site. The catheter was secured in place by two 3/0 VICRYL sutures which did not involve the site of the injury. A similar pump and catheter system containing PBS vehicle only was implanted in the middle portion of the inferior (vehicle control) abrasion site.

Two different mini pumps were used in these experiments. The first (a 2 ml pump) had a pumping rate of 10 microliters per hour and the second (a 0.2 ml pump) had a pumping rate of 0.5 microliter per hour. Each pump contained phosphate buffered saline (pH = 7.2, unless otherwise indicated), 2 milliliters in the larger pump and 0.2 milliliter in the smaller pump. The control pumps contained phosphate buffered saline alone, and the treatment pumps contained either 20 milligrams of ibuprofen (2 ml pump) or 2 milligrams of ibuprofen (0.2 ml pump).

The results seven days after the operation were as shown below in Table III. The evaluation procedure was the same as the one described in Example 4.

Table III

| Rabbit No. | Pump Size | Evaluation | |
| --- | --- | --- | --- |
| | | Ibuprofen Treatment | Vehicle Control |
| 1 | 0.2 ml | 5 | 5 |
| 2 | 0.2 ml | 4 | 5 |
| 3 | 0.2 ml | 4 | 5 |
| 4 | 2 ml | 4 | 5 |
| 5 | 2 ml | 2 | 5 |
| 6 | 2 ml | 1 | 5 |

The smaller of the two pumps gave a slight positive response in two of three rabbits, whereas the larger pump gave significant positive responses in two out of three rabbits and a slight positive response in the other.

Example 6

In order to try to better define the threshold dosage rate for ibuprofen ("IBF") in this experimental model, similar experiments were carried out with the two sizes of pumps, using different concentrations of IBF per pump. Table IV, below, sets forth the concentrations of IBF per pump and responses in this series of experiments:

Table IV

| | | | Evaluation | |
|---|---|---|---|---|
| Rabbit No. | Pump Size | Concentration of IBF. mg/ml | Treatment | Vehicle Control |
| 1 | 0.2 ml | 10 | 1 | 4 |
| 2 | 0.2 ml | 10 | 4 | 5 |
| 3 | 0.2 ml | 10 | 5 | 5 |
| 4 | 2 ml | 10 | 3 | 5 |
| 5 | 2 ml | 10 | 4 | 4 |
| 6 | 2 ml | 10 | 3 | 5 |
| 7 | 0.2 ml | 3 | 4 | 5 |
| 8 | 0.2 ml | 3 | 5 | 5 |
| 9 | 0.2 ml | 3 | 4 | 5 |
| 10 | 2 ml | 3 | 3 | 5 |
| 11 | 2 ml | 3 | 5 | 5 |
| 12 | 2 ml | 3 | 4 | 5 |
| 13 | 0.2 ml | 1 | 5 | 5 |
| 14 | 0.2 ml | 1 | 5 | 5 |
| 15 | 0.2 ml | 1 | 1 | 4 |
| 16 | 2 ml | 1 | 5 | 5 |
| 17 | 2 ml | 1 | 5 | 5 |
| 18 | 2 ml | 1 | 4 | 5 |
| 19 | 0.2 ml | 0.3 | 5 | 5 |
| 20 | 0.2 ml | 0.3 | 5 | 5 |
| 21 | 0.2 ml | 0.3 | 5 | 5 |
| 22 | 2 ml | 0.3 | 5 | 5 |
| 23 | 2 ml | 0.3 | 5 | 5 |
| 24 | 2 ml | 0.3 | 4 | 5 |

This series of experiments indicates that, in this model, the threshold dosage at which significant anti-adhesion effects began to be noted occurred when pumps containing concentrations between 1 and 3 milligrams of IBF per milliliter were used. No significant difference was noted here between the two different sized pumps.

An effective dose of a topically applied drug is normally expressed in terms of concentration of the drug in the carrier, coupled with the number of times per day the drug is applied. In the present invention, the effective dose will be dependent upon factors such as nature of specific NSAID used, nature of vehicle, nature of tissue to be treated, type of trauma, and mode of delivery (i.e., continuous delivery by catheter or a one-time application in a vehicle such as a controlled release vehicle). Therefore, no hard and fast rule can be formulated that will apply in all cases, and experiments analogous to those reported in this Example 6 will have to be performed in order to precisely define the threshold dosage for each different NSAID, for specific vehicle systems, and for specific modes of delivery. It is well within the ability of the person skilled in the art to carry out the necessary experiments to determine threshold dosages, after having read this disclosure.

Example 7

In order to determine the time period over which the anti-adhesion agent must be administered in order to have a significant anti-adhesion effect, the following series of experiments were carried out (using the experimental procedure described above in Example 5):

The 2 ml pump was used containing 10 mg/ml of IBF, and the catheter delivering the treatment solution to the site of the surgical trauma was disconnected 1, 2, 3, 4, and 5 days post-operatively. The rabbits were sacrificed 7 days post-operatively, and evaluated as above. The results are displayed in Table V, below.

Table V

| | | Evaluation | |
|---|---|---|---|
| Rabbit No. | Post-Op Day Catheter Disconnected | Ibuprofen Treatment | Vehicle Control |
| 1 | 1 | 5 | 5 |
| 2 | 1 | 4 | 4 |
| 3 | 1 | 3 | 1 |
| 4 | 2 | 4 | 4 |
| 5 | 2 | 5 | 5 |
| 6 | 2 | 1 | 1 |
| 7 | 3 | 4 | 1 |
| 8 | 3 | 1 | 3 |
| 9 | 3 | 1 | 3 |
| 10 | 4 | 4 | 5 |
| 11 | 4 | 3 | 1 |
| 12 | 4 | 1 | 1 |
| 13 | 5 | 3 | 5 |
| 14 | 5 | 4 | 5 |
| 15 | 5 | 5 | 5 |

With ibuprofen, in this model, it appears that administration of the NSAID via the 2 ml mini pump in PBS for at least three days is required in order for the drug to have significant anti-adhesion effects. With other NSAID's and/or other delivery modes, and with other types of surgical trauma, it is reasonable to expect that the minimum period of time will differ. In any given case, the minimum period of time over which the NSAID must be administered can be determined by experiments analogous to that described in this Example 7. As a general rule, in most cases, a minimum of one day is recommended, and in some cases, longer periods (e. g., up to 5 to 7 days or longer) may be required.

The NSAID active ingredient is administered to the site of surgical trauma topically. Such topical administration can be by spraying, lavage, dripping on the site, by catheter administration, or the like. The exact method of administration chosen is not critical, as long as an effective dose is administered over the critical wound healing period, which can be determined by a series of experiments analogous to that described above in Example 7. The NSAID should be administered immediately post-operatively, that is, before wound healing has begun.

Example 8

By a procedure analogous to that described above in Example 5, the 2 ml Alzet mini pump was used to deliver suprofen to the test site in the rabbits. The concentration of suprofen was 2.5 mg/ml of phosphate buffered saline (pH = 7.4). The rabbits were sacrificed 7 days post-operatively, and evaluated as above. The results are set forth in Table VI, below:

Table VI

| 2.5 mg/ml Suprofen | | |
|---|---|---|
| | Evaluation | |
| Rabbit No. | Suprofen Treatment | Vehicle Control |
| 1 | 3 | 4 |
| 2 | 4 | 3 |
| 3 | 3 | 5 |
| 4 | 4 | 5 |
| 5 | 4 | 4 |
| 6 | 3 | 5 |
| 7 | 3 | 4 |

Example 9

To a small vial was added 2.78 grams of poly(lactide-co-glycolide - 65:35), 0.30 gram suprofen, and 8 milliliters of dichloromethane. The resulting solution was added to 120 milliliters of 3% aqueous poly(vinyl alcohol) solution which was heated to 30° C. while being mechanically stirred at 500 rpm. After two hours, the microcapsules were isolated as described in Example 1, and after washing and freeze-drying, 1.61 grams of microcapsules were obtained. The microcapsules ranged in size from >10 to 250 microns and were found to contain 7.0% by weight suprofen by NMR.

Example 10

To a small vial was added 1.25 grams of poly(D,L-Lactic acid), 0.12 grams refined sesame seed oil, 0.152 gram ibuprofen, and 3 milliliters of dichloromethane. The resulting solution was added to 30 milliliters of 3% aqueous poly(vinyl alcohol) solution which was being cooled in an ice/water bath and stirred at 500 rpm. Vacuum was applied as described in Example l, and after washing and freeze-drying, l.l2 grams of microcapsules were obtained. The microcapsules ranged in size from l0 to 75 microns and were found to contain 8.7% by weight ibuprofen by NMR.

Example 11

By a procedure similar to that described above in Example 5, the 2 ml mini pump was used to deliver varying quantities of suprofen sodium (i. e., the sodium salt of suprofen) to the rabbit model. The quantities of medicament contained in the phosphate buffered saline (pH = 6.9) varied from 3.0 mg/ml down to 0.03 mg/ml. The rabbits were sacrificed seven days post-operatively and evaluated as above. The results are set forth below in Table VII:

11

## Table VII

### Suprofen, Dosage
### Response Studies

| Rabbit No. | Concentration of Suprofen, mg/ml | Evaluation | |
| --- | --- | --- | --- |
| | | Treatment | Vehicle Control |
| 1 | 3 | 1 | 1 |
| 2 | 3 | 1 | 1 |
| 3 | 3 | 1 | 1 |
| 4 | 3 | 4 | 4 |
| 5 | 3 | 1 | 1 |
| 6 | 1 | 4 | 5 |

| | | | |
|---|---|---|---|
| 7 | 1 | 1 | 1 |
| 8 | 1 | 3 | 4 |
| 9 | 1 | 1 | 5 * |
| 10 | 1 | 1 | 3 |
| | | | |
| 11 | 0.3 | 1 | 5 |
| 12 | 0.3 | 3 | 4 |
| 13 | 0.3 | 3 | 5 |
| 14 | 0.3 | 3 | 3 |
| 15 | 0.3 | 3 | 4 |
| | | | |
| 16 | 0.1 | 3 | 3 |
| 17 | 0.1 | 3 | 4 |
| 18 | 0.1 | 3 | 5 |
| 19 | 0.1 | 1 | 5 |
| 20 | 0.1 | 5 | 5 |
| | | | |
| 21 | 0.03 | 5 | 1 |
| 22 | 0.03 | 1 | 1 |
| 23 | 0.03 | 4 | 4 |
| 24 | 0.03 | 1 | 4 |
| 25 | 0.03 | 4 | 5 |

\* This rabbit exhibited slight bleeding. (In many cases, in this model, when bleeding occurs no adhesions develop.)

This series of experiments indicates that the threshold dosage at which significant anti-adhesion effects begin to be noted in this model with suprofen sodium as the NSAID occurs when pumps containing a concentration of about 0.l mg/ml of drug are used. Thus, on a weight basis, suprofen appears to be slightly more active than ibuprofen.

Example 12

By a procedure analogous to that described above in Example 5, the 2 milliliter mini pump was used to deliver varying amounts of tolmetin. (The sodium dihydrate salt of tolmetin was used.) The concentrations of drug contained in the phosphate buffered saline (pH = 7.4) varied from 3.0 mg/ml down to 0.0l mg/ml. The treatment pumps contained the concentrations of tolmetin displayed below in Table VIII, and the control pumps contained vehicle only. The rabbits were sacrificed 7 days post-operatively, with results being displayed below in Table VIII:

13

## Table VIII

### Tolmetin, Dosage
### Response Studies

| Rabbit No. | Concentration of Tolmetin, mg/ml | Evaluation | |
|---|---|---|---|
| | | Treatment | Vehicle Control |
| 1 | 3 | 1 | 1 |
| 2 | 3 | 1 | 1 |
| 3 | 3 | 1 | 5 |
| 4 | 3 | 1 | 1 |
| 5 | 3 | 1 | 3 * |
| 6 | 1 | 1 | 4 |
| 7 | 1 | 1 | 5 |
| 8 | 1 | 3 | 5 |
| 9 | 1 | 1 | 3 |
| 10 | 1 | This rabbit died ** | |

| | | | |
|---|---|---|---|
| 11 | 0.3 | 3 | 3 |
| 12 | 0.3 | 1 | 3 * |
| 13 | 0.3 | 1 | 3 |
| 14 | 0.3 | 3 | 5 |
| 15 | 0.3 | 3 | 4 |
| 16 | 0.1 | 3 | 5 |
| 17 | 0.1 | 1 | 5 |
| 18 | 0.1 | 1 | 5 |
| 19 | 0.1 | 1 | 5 |
| 20 | 0.1 | This rabbit died (snuffles)*** | |
| 21 | 0.03 | 1 | 1 |
| 22 | 0.03 | 1 | 5 |
| 23 | 0.03 | 3 | 5 |
| 24 | 0.03 | 1 | 5 |
| 25 | 0.03 | 1 | 4 |
| 26 | 0.01 | 5 | 5 |
| 27 | 0.01 | 1 | 5 |
| 28 | 0.01 | 1 | 5 |
| 29 | 0.01 | 1 | 4 |
| 30 | 0.01 | 1 | 1 |

----------------------------------------------------------------

\* Slight bleeding occurred.

\*\* The cause of death was unknown, but there was no suggestion that it was drug-related.

\*\*\* "Snuffles" is a virus-caused upper respiratory disease that affects rabbits. There is no suggestion that it was drug-related.

----------------------------------------------------------------

As can be seen from the data presented in Table VIII, tolmetin appears to be more effective, on a weight basis, than either ibuprofen or suprofen in this rabbit model experiment. The threshold dosage, as indicated by the results from this experiment, was apparently less than the dosage administered to Rabbit Nos. 26 - 30, wherein the concentration of drug was 0.0l mg/ml.

Example l3

15

The following is a typical preparation of a liposome (MLV) containing an NSAID:
(In this preparation, all materials and equipment used are sterile and pyrogen-free)

a) Preparation of lipid film

L-alpha-distearoyl phosphatidylcholine ("DSPC"), I.2I gm., and 0.29 gm. cholesterol (molar ratio of DSPC to cholesterol is 2:I), are dissolved in 45 ml. of chloroform. The resulting solution is divided into nine 5 ml portions, and each such portion is placed in a I00 ml flask. The solvent is evaporated from each flask using a rotary vacuum evaporator. Sterility is maintained by attaching a 0.22 micron Millex filter to the air intake of the evaporator prior to flask removal. Sterile septa are placed on the flasks after solvent evaporation. The surface of each septum is wiped with 70% alcohol, and a I9 gauge sterile needle affixed to a 0.22 micron filter is passed through each septum. All flasks are then placed in a large vacuum desiccator and kept there overnight. Each flask contains about I67 mg. of lipid.

b) Preparation of NSAID solution

The sodium salt of ibuprofen (Na-IBF), 0.202 gm., is dissolved in 40 ml of sterile, pyrogen-free water. The solution is then passed through a 0.22 micron Millex filter.

c) Preparation of MLV containing Na-IBF

3.9 MI of the Na-IBF solution is injected into each flask containing lipid film. The flasks are vortex-stirred for 40 to 60 minutes in a 65° C. water bath under nitrogen. (The nitrogen purge is first passed through a 0.22 micron filter.) To the contents of each flask are added sterile, pyrogen-free phosphate buffered saline, and the flasks are centrifuged for 6 to I0 minutes at I5,000 rpm. This washing procedure is repeated for a total of five times to remove unencapsulated NSAID. The contents of the flasks are then combined and PBS (5mM $PO_4$ in 0.I5 NaCl) is added to a total of 32 ml. The liposome suspension thus produced comprises MLV's of about I micron in size suspended in the PBS. It is storage stable for a period of several months, but for long term storage is preferably dehydrated and remixed with sterile, pyrogen-free water just before use.

To prepare drug-free controls, the procedure is repeated substituting pure water for the water/Na-IBF solution.

The procedure is repeated using (a) the free-acid form of IBF, and (b) the sodium salt of tolmetin. Similar results are obtained and liposome MLV's containing IBF or sodium tolmetin are produced.

MLV's containing IBF or tolmetin, and produced as described herein, were found to have particle sizes of about I-I/2 to 2 microns (by optical microscope) and about 5 to 8 microns (by Coulter counter).

Analogous procedures would be employed to produce vesicles from phosphatidylcholines in which the fatty acid moieties were derived from other fatty acids, e.g., $C_{12}$ to $C_{24}$ fatty acids. $C_{14}$ to $C_{20}$ saturated fatty acids are preferred.

Example I4

This experiment evaluated the efficacy of a liposome/ibuprofen combination to combat post-surgical adhesions. The procedure used was similar to that described above in Example 4, except that the untreated control sites in those rabbits that were given the liposome/ibuprofen treatment were on the left-lateral peritoneal side-wall instead of being sited I.5 to 2.0 centimeters inferior to the treatment sites in the right-lateral peritoneal side wall, and the wounding procedure described in Example 5 was used.

The liposomes used in this example were DSPC/cholesterol MLV's (L-alpha-distearoyl phosphatidylcholine/cholesterol multilamellar vesicles), prepared in a manner analogous to that described above in Example I3. The treatment mixture consisted of 3I milliliters containing I200 milligrams of MLV and 35 milligrams of the free acid form of ibuprofen, suspended in 5mM phosphate buffered saline. The vehicle control had the same composition, except that the ibuprofen was omitted. Each rabbit received I0 ml of suspension, which amounted to 3.5 mg of ibuprofen per rabbit. The treatment and vehicle control suspensions were dripped on the traumatized sites, as described above in Example 4. Three rabbits received the liposome/ibuprofen treatment on the right side-wall site with the other site being untreated, and three rabbits received a vehicle control treatment (i. e., liposome without ibuprofen) on the right side-wall site with no treatment on the other site. The rabbits were sacrificed 7 days post-operatively, and evaluated as described above. The results are displayed below in Table IX:

Table IX

| Liposome/Ibuprofen Studies | | | |
| --- | --- | --- | --- |
| | | Site | |
| Rabbit No. | Ibuprofen | Treated | Untreated |
| 1 | yes | 1 | 1 |
| 2 | yes | 1 | 1 |
| 3 | yes | 1 | 4 |
| 4 | no | 2 | 4 |
| 5 | no | 5 | 5 |
| 6 | no | 1 | 5 |

The experiment was repeated in exactly the same fashion, with the results as displayed below in Table X:

Table X

| | | Site | |
| --- | --- | --- | --- |
| Rabbit No. | Ibuprofen | Treated | Untreated |
| 1 | yes | 1 | 1 |
| 2 | yes | 1 | 1 |
| 3 | yes | 1 | 4 |
| 4 | no | 5 | 4 |
| 5 | no | 3 | 4 |
| 6 | no | 1 | 1* |

* This rabbit exhibited slight bleeding.

In the experiments reported above in Tables IX and X, the rabbits receiving treatment by the ibuprofen/liposome combination were virtually free of adhesions, even on the sides that received no direct application of medicament. This is considered to be indicative of the fact that the medicament can migrate in the peritoneal cavity as a result of circulation of peritoneal fluid. Also, by gross observation, no tissue reaction or granulomas were found at the treated sites.

Example 15

In this series of experiments, the procedure of Example 14 was repeated, with the following modifications:

The liposomes that were used were DSPC/cholesterol MLV's prepared as described above in Example 13, and which contained Na-IBF. The control MLV's (two batches - "MLV-I" and "MLV-2") contained no drug. The drug-containing liposomes were mixed with the control liposomes in varying proportions to obtain MLV's that contained varying amounts of NSAID. In Table XI, below, there is displayed the compositions of the drug-containing and the control liposomes:

## Table XI

## Liposome Composition

|  | Vol, ml | Lipid, mg/ml | Total Lipid, mg | Drug, mg/ml | Drug/Lipid, (by weight) |
|---|---|---|---|---|---|
| **Control** | | | | | |
| MLV-1 | 50 | 33 | 1675 | N/A | N/A |
| MLV-2 | 50 | 34 | 1710 | N/A | N/A |
| **IBUPROFEN** | | | | | |
| MLV | 50 | 33 | 1675 | 0.98 (49 gm) | 0.029 |

In Table XII, below, there is displayed the proportions of the drug-containing and control liposomes that were mixed together and then administered to the test rabbits in the manner described above in Example I4, along with the results of the evaluations of the rabbits seven days post operatively:

Table XII

| Na-IBF/MLV Evaluations | | | | |
|---|---|---|---|---|
| Rabbit No. | IBF MLV. ml | Control MLV. ml | Evaluation | |
| | | | Treatment Site | Control Site |
| 1 | 10 | 0 | 1 | 1 |
| 2 | 10 | 0 | 3 | 3 |
| 3 | 10 | 0 | 1 | 1 |
| 4 | 3 | 7 | 1 | 1 |
| 5 | 3 | 7 | 1 | 4 |
| 6 | 3 | 7 | 1 | 5 |
| 7 | 3 | 7 | 1 | 4 |
| 8 | 1 | 9 | 1 | 1 |
| 9 | 1 | 9 | 1 | 1 |
| 10 | 1 | 9 | 1 | 1 |
| 11 | 1 | 9 | 1 | 1 |
| 12 | 0.3 | 9.7 | 1 | 3 |
| 13 | 0.3 | 9.7 | 1 | 3 |
| 14 | 0 | 10 | 4 | 5 |
| 15 | 0 | 10 | 5 | 3 |
| 16 | 0 | 10 | 3 | 3 |
| 17 | 0 | 10 | 4 | 5 |
| 18 | 0 | 10 | 3 | 3 |

Example 16

By a procedure analogous to that described above in Example 14, aqueous compositions including a surface active agent and the free acid form of tolmetin were applied to the treatment sites in the rabbit model. The aqueous compositions comprised various concentrations of "Tween 80", an ethoxylated sorbitan mono-oleate, in triple distilled water, and tolmetin at a concentration of either 1 or 2 mg/ml. The solutions were sterilized by passing them through a 0.22 micron filter. The tables below display the concentration of tolmetin and Tween 80, and the evaluations of the adhesions seven days post-operatively. In each case, 10 ml of the solution was dripped on the treatment site.

Table XIII

| 5 Wt. % Tween 80 1 mg/ml Tolmetin | | |
|---|---|---|
| Rabbit No. | Evaluation | |
| | Treatment Site | Control Site |
| 1 | 1 | 5 |
| 2 | 1 | 1 |
| 3 | 1 | 1 (Bleeding) |
| 4 | 1 | 1 |
| 5 (Control*) | 3 | 3 |
| 6 | 1 | 3 |
| 7 | 1 | 4 |
| 8 | 3 | 5 |
| 9 | 1 | 1 |
| 10 (Control*) | 4 | 3 |

* The control rabbits received no treatment.

Table XIV

| 20 Wt. % Tween 80 2 mg/ml Tolmetin | | |
|---|---|---|
| Rabbit No. | Evaluation | |
| | Treatment Site | Control Site |
| 1 | 1 | 5 |
| 2 | 3 | 5 |
| 3 | 1 | 4 |
| 4 | 3 | 1 |
| 5 (Control*) | 4 | 4 |
| 6 | 1 | 3 |
| 7 | 3 | 5 |
| 8 | 1 | 1 |
| 9 | 1 | 1 |
| 10 (Control*) | 5 | 5 |

* No Treatment

# EP 0 225 162 B1

Table XV

| 20 Wt. % Tween 80 2 mg/ml Tolmetin | | |
|---|---|---|
| Rabbit No. | Evaluation | |
| | Treatment Site | Control Site |
| 1 | 1 | 5 |
| 2 | 1 | 1 (Bleeding) |
| 3 | 1 | 5 |
| 4 | 1 | 5 |
| 5 | 1 | 4 |
| 6 | 1 | 4 |
| 7 | 1 | 4 |
| 8 | 1 | 1 |

Table XVI

| 1 mg/ml Tolmetin Sodium (1) No Tween | | |
|---|---|---|
| Rabbit No. | Evaluation | |
| | Treatment Site | Control Site |
| 1 | 3 | 5 |
| 2 | 4 | 3 |
| 3 | 1 | 3 |
| 4 | 3 | 4 |
| 5 | 3 | 3 |
| 6 | 4 | 3 |
| 7 | 1 | 3 |
| 8 | 3 | 5 |
| 9 (Control[2]) | 4 | 3 |

(1) The sodium salt was used because the free acid form is quite insoluble in pure water.
(2) Water only: no Tolmetin

The free acid form of tolmetin, which is quite insoluble in water, was maintained in aqueous solution or colloidal suspension by the surface active agent.

Referring again to the question of the effective dose of NSAID in accordance with this invention, while no hard and fast numbers can be presented that will be applicable to all cases, the examples presented above can be referred to as a guide to determine the order of magnitude of drug to employ in certain cases. In phosphate buffered saline administered continually via the Alzet osmotic mini pump, the following dosages were found to be effective:

ibuprofen, 3 mg/ml, at a rate of 0.5 microliter/hr.(from Ex.6);

suprofen, 0.l mg/ml, at a rate of l0 microliters/hr. (from Ex. ll); and

tolmetin, 0.0l mg/ml, at a rate of l0 microliters/hr. (from Ex. l2).

Expressed in terms of mg/kg/day, and in terms of $mg/day/cm^2$ of traumatized tissue, these numbers are the following:

ibuprofen    - l8 $\times$ l0$^{-3}$ mg/kg/day
            2.4 $\times$ l-$^{-3}$ mg/day/cm$^2$

suprofen    - l2 $\times$ l0$^{-3}$ mg/kg/day
            l.6 $\times$ l0$^{-3}$ mg/day/cm$^2$

tolmetin    - l.2 $\times$ l0$^{-3}$ mg/kg/day
            0.l6 $\times$ l0$^{-3}$ mg/day/cm$^2$

These calculations are carried out as follows (using the ibuprofen numbers as illustrative): 0.5

20

microliter/hr. of solution containing 3 mg/ml equals $0.5 \times 10^{-6}$ liter/hr. $\times$ (3 mg/$10^{-3}$ liter). This reduces to $0.5 \times 3 \times 10^{-6}/10^{-3} = 1.5 \times 10^{-3}$ mg/hr.

This equals $36 \times 10^{-3}$ mg/day or $18 \times 10^{-3}$ mg/kg/day (each rabbit weighs about 2 kg).

The area of traumatized tissue is about 15 cm$^2$, so the dose expressed in terms of mg/day/cm$^2$ = 36/15 $\times 10^{-3}$ mg/day/cm$^2$. or $2.4 \times 10^{-3}$ mg/day/cm$^2$.

Compared with the minimum recommended dose of 2.5 mg/kg/day (from the Singer patent cited above) for systemically administered ibuprofen, the effective dose for topically administered ibuprofen may be two to three orders of magnitude lower. Obviously, this greatly reduced dosage significantly reduces the chances for undesired side effects.

The minimum effective dose using an MLV or an aqueous composition containing a surface active agent as a carrier has apparently not been approached. However, it is noted from Example 15, Rabbit Nos. 12-13, that a total dose of about 0.3 mg of ibuprofen in an MLV carrier was effective. This number was calculated as follows:

0.98 mg/ml $\times$ 0.3 ml/0.7 ml, equals 0.0278 mg/ml. 10 ml of this preparation was administered, so the total dosage was 0.3 mg or 0.15 mg/kg or 0.02 mg/cm$^2$. It would appear that an even lower concentration would be effective in this model.

In the Tween 80 solutions, the smallest amount of tolmetin applied was 10 mg (from the data presented in Table XIII). It would appear that an even smaller dosage administered in this manner would be effective. A reasonable extrapolation of the data presented herein is that a minimum effective concentration for an NSAID preparation applied in a single dose would be between about 0.025 mg and 5 mg of NSAID per ml of total vehicle. (By "total vehicle" is meant an organic vehicle such as MLV or Tween 80 plus diluent such as water or PBS.)

Similarly, for an NSAID preparation that is administered continuously, as by catheter, minimum effective concentrations of NSAID in total vehicle will usually be found within the range of from about 0.01 to about 10 mg/ml.

Example 17

In this series, the uterine horn of New Zealand white female rabbits was used as the model for adhesion development. It is believed that the trauma induced in this type of surgical procedure is more apt to produce severe adhesions than any trauma ordinarily associated with surgery, and therefore this is a very severe test for evaluating the efficacy of a medicament in inhibiting the formation of post-surgical adhesions.

The rabbits were anaesthesized using acelepromazine and ketamine, and then underwent a lower median laparotomy incision. Then, the serosal surface of both uterine horns were abraded by grasping them with a gauze surgical sponge and pulling them away from the uterus until punctate bleeding developed.

Immediately after the uterine horns were traumatized as described above, varying quantities of MLV liposome (prepared by a procedure analogous to that described above in Example 13) containing the sodium salt of tolmetin were dripped on the traumatized site, and the rabbits were then closed. Seven days postoperatively, the rabbits were sacrificed and the development of adhesions was evaluated, with the results discussed below.

The liposomes were suspended in PBS, at a concentration of 40 mg. of liposome per ml of suspension and 1.46 mg. of tolmetin per ml of suspension.

The results of the evaluation were as follows, with the quantities indicated being the volume of liposome suspension dripped on the site of surgical trauma:

10 ml       - Scant adhesion

3 ml        - Scant adhesion, but a few more than with the 10 ml dose. The adhesions were filmy

1 ml        - Mild adhesions

Control     - (no medicament)- Severe adhesions; essentially unable to open the rabbit without tearing adhesions which developed between the uterus and the bowel and  between the uterus and the anterior peritoneal wall.

The above-described study was repeated several times with the liposome/tolmetin combination, with essentially the same results.

When the free acid form of tolmetin contained in 15 milliliters of aqueous Tween 80 (5 weight per cent Tween 80, 1 mg/ml of tolmetin) was substituted for the tolmetin/liposome suspension in the double uterine horn model study, the formation of post-surgical adhesions was substantially prevented.

**Claims**

EP 0 225 162 B1

1. A pharmaceutical composition for topical application comprising a topically effective amount of a non-steroidal anti-inflammatory drug (NSAID) or a pharmaceutically acceptable salt or ester thereof as the active ingredient in a topically applicable pharmaceutically acceptable carrier, provided that the NSAID is not aspirin, wherein said carrier is a controlled release carrier which releases the active ingredient in an effective amount over a period of time of from about one to seven days.

2. The composition of claim 1 which is adapted to be administered in a single dose, and wherein the concentration of active ingredient in said composition is from 0.025 to 5 milligrams per millilitre.

3. The composition of claim 1 which is adapted to be administered continually over a period of time, and wherein the concentration of active ingredient in said composition is from 0.01 to 10 milligrams per millilitre.

4. A pharmaceutical composition adapted for topical adminstration in a single dose comprising a topically effective amount of a non-steroidal anti-inflammatory drug (NSAID) or a pharmaceutically acceptable salt or ester thereof as the active ingredient in a topically applicable pharmaceutically acceptable carrier suitable for topical administration to a site of surgical trauma, provided that the NSAID is not aspirin and wherein the concentration of active ingredient in said composition is from 0.025 to 5 milligrams per millilitre.

5. A pharmaceutical composition adapted for topical adminstration continually over a period of time comprising a topically effective amount of a non-steroidal anti-inflammatory drug (NSAID) or a pharmaceutically acceptable salt or ester thereof as the active ingredient in a topically applicable pharmaceutically acceptable carrier suitable for topical administration to a site of surgical trauma, provided that the NSAID is not aspirin and wherein the concentration of active ingredient in said composition is from 0.01 to 10 milligrams per millilitre.

6. The composition of either of claims 4 and 5 wherein the carrier is a controlled release carrier which releases the active ingredient in an effective amount over a period of time of from about one to seven days.

7. The composition of any one of claims 1 to 6 wherein the active ingredient is ibuprofen or a pharmaceutically acceptable salt or ester thereof.

8. The composition of any one of claims 1 to 6 wherein the active ingredient is suprofen or a pharmaceutically acceptable salt or ester thereof.

9. The composition of any one of claims 1 to 6 wherein the active ingredient is tolmetin or a pharmaceutically acceptable salt or ester thereof.

10. The composition of any one of claims 1 to 3 and 6 to 9 wherein said carrier is a phospholipid.

11. The composition of claim 10 wherein said phospholipid is a phospholipid vesicle.

12. The composition of claim 11 wherein the phospholipid vesicle is a multilamellar vesicle.

13. The composition of claim 12 wherein the vesicle has a size of from 1 to 10 micrometers.

14. The composition of claim 12 or claim 13 wherein the vesicle comprises phosphatidyl choline wherein the fatty acid moieties are derived from a single fatty acid.

15. The composition of claim 12 or claim 13 wherein the vesicle comprises a mixture of a phosphatidyl choline and cholesterol.

16. The composition of any one of claims 1 to 3 and 6 to 9 wherein said carrier is an absorbable polymer.

17. The composition of claim 16 wherein said absorbable polymer is a homopolymer or copolymer of lactic acid, glycolic acid, their cyclic dimer esters, or p-dioxanone.

22

**18.** The composition of claim 17 wherein said polymer is in the form of microcapsules.

**19.** The composition of claim 18 wherein said microcapsules include lecithin in an amount sufficient to enhance the ability of said microcapsules to adhere to the site of surgical trauma.

**20.** The composition of any one of claims 4, 5 and 7 to 9, wherein the carrier is an aqueous composition including a surface active agent.

**21.** The composition of claim 20 wherein the aqueous composition comprises an aqueous solution of ethoxylated sorbitan mono-oleate.

**22.** Use of a non-steroidal anti-inflammatory drug (NSAID) or a pharmaceutically acceptable salt or ester thereof in the production of a topically applicable medicament for topical use in inhibiting the formation of post-surgical adhesions in mammals, provided that the NSAID is not aspirin.

**23.** Use of a non-steroidal anti-inflammatory drug or a pharmaceutically acceptable salt or ester thereof in the production of a composition according to any one of claims 1 to 21 for topical use in inhibiting the formation of postsurgical adhesions in mammals, provided that the NSAID is not aspirin.

**24.** A method of making a pharmaceutical composition according to any one of claims 1 to 21 which comprises admixing a non-steroidal anti-inflammatory drug (NSAID) other than aspirin with a topically applicable pharmaceutically acceptable carrier, suitable for topical administration to a site of surgical trauma and as defined in any one of claims 1, 4 and 5, to produce a composition according to any one of claims 1 to 21.

**Revendications**

**1.** Composition pharmaceutique pour application locale comprenant une quantité localement efficace d'un médicament anti-inflammatoire non-stéroïdique (NSAID) ou d'un de ses sels ou esters pharmaceutiquement acceptables comme ingrédient actif dans un support pharmaceutiquement acceptable localement applicable, à condition que le NSAID ne soit pas de l'aspirine, où ledit support est un support à libération contrôlée qui libère l'ingrédient actif en une quantité efficace pendant une durée allant d'environ un à sept jours.

**2.** Composition de la revendication 1 qui est adaptée pour être administrée en une seule dose, et où la concentration de l'ingrédient actif dans ladite composition est de 0,025 à 5 miligrammes par millilitre.

**3.** Composition de la revendication 1 qui est adaptée pour être administrée de façon continue pendant une certaine durée, et où la concentration d'ingrédient actif dans ladite composition est de 0,01 à 10 milligrammes par millilitre.

**4.** Composition pharmaceutique adaptée pour l'administration locale en une seule dose comprenant une quantité localement eficace d'un médicament anti-inflammatoire non-stéroïdique (NSAID) ou d'un de ses sels ou esters pharmaceutiquement acceptables comme ingrédient actif dans un support pharmaceutiquement acceptable localement applicable approprié à l'administration locale en un site de traumatisme chirurgical, à condition que le NSAID ne soit pas de l'aspirine, et où la concentration en ingrédient actif dans ladite composition est de 0,025 à 5 milligrammes par millilitre.

**5.** Composition pharmaceutique adaptée pour l'administration locale de façon continue pendant une certaine durée comprenant une quantité localement efficace d'un médicament anti-inflammatoire non-stéroïdique (NSAID) ou d'un de ses sels ou esters pharmaceutiquement acceptables comme ingrédient actif dans un support pharmaceutiquement acceptable localement applicable approprié à l'administration locale à un site de traumatisme chirurgical, à condition que le NSAID ne soit pas de l'aspirine, et où la concentration d'ingrédient actif dans ladite composition est de 0,01 à 10 milligrammes par millilitre.

**6.** Composition de l'une quelconque des revendications 4 et 5 dans laquelle le support est un support à libération contrôlée qui libère l'ingrédient actif en une quantité efficace pendant une durée allant

EP 0 225 162 B1

d'environ un à sept jours.

7. Composition de l'une quelconque des revendications 1 à 6 dans laquelle l'ingrédient actif est l'ibuprofène ou un de ses sels ou esters pharmaceutiquement acceptables.

8. Composition de l'une quelconque des revendications 1 à 6 dans laquelle l'ingrédient actif est le suprofène ou un de ses sels ou esters pharmaceutiquement acceptables.

9. Composition de l'une quelconque des revendications 1 à 6 dans laquelle l'ingrédient actif est la tolmétine ou un de ses sels ou esters pharmaceutiquement acceptables.

10. Composition de l'une quelconque des revendications 1 à 3 et 6 à 9 dans laquelle ledit support est un phospholipide.

11. Composition de la revendication 10 dans laquelle ledit phospholipide est une vésicule de phospholipide.

12. Composition de la revendication 11 dans laquelle la vésicule de phospholipide est une vésicule multi-lamellaire.

13. Composition de la revendication 12 dans laquelle la vésicule a une taille de 1 à 10 micromètres.

14. Composition de la revendication 12 ou de la revendication 13, dans laquelle la vésicule comprend une phosphatidyl-choline dans laquelle les parties acide gras sont dérivées d'un seul acide gras.

15. Composition de la revendication 12 ou de la revendication 13 dans laquelle la vésicule comprend un mélange d'une phosphatidyl-choline et de cholestérol.

16. Composition de l'une quelconque des revendications 1 à 3 et 6 à 9 dans laquelle ledit support est un polymère absorbable.

17. Composition de la revendication 16 dans laquelle ledit polymère absorbable est un homopolymère ou copolymère de l'acide lactique, de l'acide glycolique, de leurs esters dimères cycliques, ou de la p-dioxanone.

18. Composition de la revendication 17 dans laquelle ledit polymère est sous la forme de microcapsules.

19. Composition de la revendication 18 dans laquelle lesdites microcapsules comprennent de la lécithine en une quantité suffisante pour accroître l'aptitude desdites microcapsules à adhérer au site du traumatisme chirurgical.

20. Composition de l'une quelconque des revendications 4, 5 et 7 à 9 dans laquelle le support est une composition aqueuse incluant un agent tensio-actif.

21. Composition de la revendication 20 dans laquelle la composition aqueuse comprend une solution aqueuse de mono-oléate de sorbitan éthoxylé.

22. Application d'un médicament anti-inflammatoire non-stéroïdique (NSAID) ou d'un de ses sels ou esters pharmaceutiquement acceptables à la production d'un médicament localement applicable pour l'utilisation locale afin d'inhiber la formations d'adhérences postchirurgicales chez les mammifères, à condition que le NSAID ne soit pas de l'aspirine.

23. Application d'un médicament anti-inflammatoire non-stéroïdique ou d'un de ses sels ou esters pharmaceutiquement acceptables à la production d'une composition selon l'une quelconque des revendications 1 à 21 pour application locale afin d'inhiber la formation d'adhérences post-chirurgicales chez les mammifères, à condition que le NSAID ne soit pas de l'aspirine.

24. Procédé de préparation d'une composition pharmaceutique selon l'une quelconque des revendications

24

1 à 21 dans lequel on mélange un médicament anti-inflammatoire non-stéroïdique (NSAID) autre que l'aspirine avec un support pharmaceutiquement acceptable localement applicable, approprié pour l'administration locale au site d'un traumatisme chirurgical et tel que défini dans l'une quelconque des revendications 1, 4 et 5, pour produire une composition selon l'une quelconque des revendications 1 à 21.

**Patentansprüche**

1. Pharmazeutische Zusammensetzung für die topische Anwendung, enthaltend eine topisch wirksame Menge eines nicht-steroiden, entzündungshemmenden Medikaments (NSEM) oder eines pharmazeutisch verwendbaren Salzes oder Esters davon als Wirkstoff in einem topisch anwendbaren, pharmazeutisch verwendbaren Träger, mit der Maßgabe, daß das NSEM nicht Aspirin ist, wobei der genannte Träger ein Träger mit kontrollierter Freigabe ist, der den Wirkstoff in einer wirksamen Menge über eine Zeit von etwa einem bis sieben Tagen freisetzt.

2. Zusammensetzung nach Anspruch 1, die so angepaßt ist, daß sie als Einzeldosis verabreicht werden kann, und in der die Konzentration an Wirkstoff in der Zusammensetzung von 0,025 bis 5 mg je ml beträgt.

3. Zusammensetzung nach Anspruch 1, die so angepaßt ist, daß sie kontinuierlich über eine Zeitdauer verabreicht werden kann, und in der die Konzentration des Wirkstoffs in der Zusammensetzung von 0,01 bis 10 mg je ml beträgt.

4. Pharmazeutische Zusammensetzung für die topische Verabreichnug in einer Einzeldosis, enthaltend eine topisch wirksame Menge eines nicht-steroiden, entzündungshemmenden Medikaments (NSEM) oder eines pharmazeutisch verwendbaren Salzes oder Esters davon als Wirkstoff in einem topisch anwendbaren, pharmazeutisch verwendbaren Träger, der für die topische Verabreichung an eine Stelle eines operativen Traumas geeignet ist, mit der Maßgabe, daß das NSEM nicht Aspirin ist, und in der die Konzentration an Wirkstoff in der Zusammensetzung von 0,025 bis 5 mg je ml beträgt.

5. Pharmazeutische Zusammensetzung für die topische kontinuierliche Verabreichung über eine Zeitdauer, enthaltend eine topisch wirksame Menge eines nicht-steroiden, entzündungshemmenden Medikaments (NSEM) oder eines pharmazeutisch verwendbaren Salzes oder Esters davon als Wirkstoff in einem topisch anwendbaren, pharmazeutisch verwendbaren Träger, der für die topische Verabreichung an eine Stelle eines operativen Traumas geeignet ist, mit der Maßgabe, daß das NSEM nicht Aspirin ist, und in der die Konzentration an Wirkstoff in der Zusammensetzung von 0,01 bis 10 mg je ml beträgt.

6. Zusammensetzung nach Anspruch 4 oder 5, in welcher der Träger ein Träger mit kontrollierter Freigabe ist, der den Wirkstoff in einer wirksamen Menge über eine Zeit von etwa einem bis sieben Tagen freisetzt.

7. Zusammensetzung nach einem der Ansprüche 1 bis 6, in der der Wirkstoff Ibuprofen oder ein pharmazeutisch verwendbares Salz oder ein pharmazeutisch verwendbarer Ester davon ist.

8. Zusammensetzung nach einem der Ansprüche 1 bis 6, in der der Wirkstoff Suprofen oder ein pharmazeutisch verwendbares Salz oder ein pharmazeutisch verwendbarer Ester davon ist.

9. Zusammensetzung nach einem der Ansprüche 1 bis 6, in der der Wirkstoff Tolmetin oder ein pharmazeutisch verwendbares Salz oder ein pharmazeutisch verwendbarer Ester davon ist.

10. Zusammensetzung nach einem der Ansprüche 1 bis 3 und 6 bis 9, in der der genannte Träger ein Phospholipid ist.

11. Zusammensetzung nach Anspruch 10, in der das genannte Phospholipid ein Phospholipid-Bläschen ist.

12. Zusammensetzung nach Anspruch 11, in der das Phospholipid-Bläschen ein mehrblättriges Bläschen ist.

**13.** Zusammensetzung nach Anspruch 12, in der das Bläschen eine Größe von 1 bis 10 m hat.

**14.** Zusammensetzung nach Anspruch 12 oder 13, in der das Bläschen Phosphatidylcholin enthält, in dem die Fettsäure-Reste sich von einer einzigen Fettsäure ableiten.

**15.** Zusammensetzung nach Anspruch 12 oder 13, in der das Bläschen ein Gemisch von Phosphatidylcholin und Cholesterin enthält.

**16.** Zusammensetzung nach einem der Ansprüche 1 bis 3 und 6 bis 9, in der der genannte Träger ein absorbierbares Polymer ist.

**17.** Zusammensetzung nach Anspruch 16, in der das genannte absorbierbare Polymer ein Homopolymer oder Copolymer von Milchsäure, Glykolsäure, ihren cyclischen dimeren Estern oder p-Dioxanon ist.

**18.** Zusammensetzung nach Anspruch 17, in der das genannte Polymer in Form von Mikrokapseln vorliegt.

**19.** Zusammensetzung nach Anspruch 18, in der die genannten Mikrokapseln Lecithin in einer ausreichenden Menge enthalten, um die Fähigkeit der Mikrokapseln zu erhöhen, an der Stelle des operativen Traumas zu haften.

**20.** Zusammensetzung nach einem der Ansprüche 4,5 und 7 bis 9, in der der genannte Träger eine wäßrige, ein oberflächenaktives Mittel enthaltende Zusammensetzung ist.

**21.** Zusammensetzung nach Anspruch 20, in der die wäßrige Zusammensetzung eine wäßrige Lösung von ethoxyliertem Sorbitan-monoleat ist.

**22.** Verwendung eines nicht-steroiden, entzündungshemmenden Medikaments (NSEM) oder eines pharmazeutisch verwendbaren Salzes oder Esters davon zur Herstellung eines topisch anwendbaren Medikaments zur topischen Verwendung bei der Hemmung der Bildung von post-operativen Adhäsionen bei Säugetieren, mit der Maßgabe, daß das NSEM nicht Aspirin ist.

**23.** Verwendung eines nicht-steroiden, entzündungshemmenden Medikaments (NSEM) oder eines pharmazeutisch verwendbaren Salzes oder Esters davon zur Herstellung einer Zusammensetzung nach einem der Ansprüche 1 bis 21 zur topischen Verwendung bei der Hemmung der Bildung von post-operativen Adhäsionen bei Säugetieren, mit der Maßgabe, daß das NSEM nicht Aspirin ist.

**24.** Verfahren zur Herstellung einer pharmazeutischen Zusammensetzung nach einem der Ansprüche 1 bis 21, umfassend das Vermischen eines nicht-steroiden entzündungshemmenden Medikaments (NSEM), Aspirin ausgenommen, mit einem topisch anwendbaren, pharmazeutisch verwendbaren Träger, der für die topische Verabreichung an einer Stelle eines operativen Traumas geeignet und in einem der Ansprüche 1, 4 und 5 definiert ist, zur Herstellung einer Zusammensetzung nach einem der Ansprüche 1 bis 21.